**Europäisches Patentamt**

**European Patent Office**

**Office européen des brevets**

(19)

(11) **EP 1 467 274 A2**

(12) **EUROPEAN PATENT APPLICATION**

(43) Date of publication:
   **13.10.2004 Bulletin 2004/42**

(51) Int Cl.$^7$: **G06F 1/00**

(21) Application number: **04252039.5**

(22) Date of filing: **06.04.2004**

| | |
|---|---|
| (84) Designated Contracting States:<br>**AT BE BG CH CY CZ DE DK EE ES FI FR GB GR HU IE IT LI LU MC NL PL PT RO SE SI SK TR**<br>Designated Extension States:<br>**AL HR LT LV MK** | (71) Applicant: **SHARP KABUSHIKI KAISHA**<br>**Osaka 545-8522 (JP)** |
| (30) Priority: **08.04.2003 JP 2003103739**<br>**29.05.2003 JP 2003152234** | (72) Inventor: **Ohyama, Shigeo**<br>**Nara-shi, Nara 631-0072 (JP)**<br><br>(74) Representative: **Brown, Kenneth Richard et al**<br>**R.G.C. Jenkins & Co.**<br>**26 Caxton Street**<br>**London SW1H 0RJ (GB)** |

(54) **Scrambler circuit**

(57)     This invention is intended to provide a scrambler circuit capable of realizing a data processing device or an IC card having high security enough to prevent information in a memory or information on a bus from being decrypted. The scrambler circuit has to-be-processed data divided into two data blocks (B1 and B0) and processed data divided into two data blocks (B1' and B0'), and includes a first scrambler unit (231) that conducts first scrambling to the data block (B1) and that outputs first intermediate data, a first arithmetic unit (233) that performs an exclusive OR operation between the data block (B0) and the first intermediate data and that outputs the data block (B1'), a second scrambler unit (232) that conducts second scrambling to the data block (B1') and that outputs second intermediate data, and a second arithmetic unit (234) that performs an exclusive OR operation between the second intermediate data and the data block (B1) and that outputs the data block (B0').

FIG. 3A

EP 1 467 274 A2

**Description**

BACKGROUND OF THE INVENTION

1. Field of the Invention

The present invention relates to a security technique for a data processing device. More specifically, the present invention relates to a security technique for a data processing device constituted by a semiconductor integrated circuit, for protecting internal information of the semiconductor integrated circuit from being read or falsified due to probing by a malicious intruder, and from being read by a separation analysis to the semiconductor integrated circuit.

2. Description of the Related Art

To ensure high security is required for a one-chip microcontroller which stores secret information such as individual information and which is used in a system such as an IC card that processes the secret information. In order to prevent internal information of the one-chip microcontroller from being read or programmed due to an intruder's attack (intruder's secret information analysis behavior), it is necessary to protect the information.

Conventionally, information is protected from an analysis behavior by irregularly connecting wirings of an address bus and a data bus for transmitting signals between logic circuits including memories and the one-chip microcontroller, and by making it difficult to specify a function of each signal line. However, recent analysis techniques have enhanced practically enough to specify the signal line by a separation analysis.

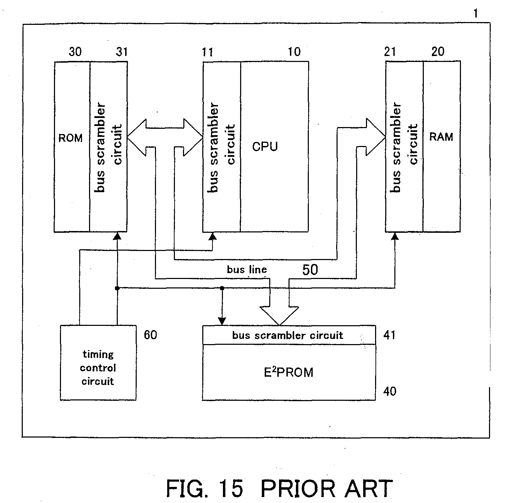

To solve this disadvantage, Japanese Unexamined Patent Publication No. 11-203237 discloses a technique for performing bus scrambling by regularly changing an order of signals on a bus. Fig. 15 illustrates the technique disclosed by 11-203237. In Fig. 15, reference symbol 1 denotes a semiconductor integrated circuit. The semiconductor integrated circuit 1 includes therein functional blocks such as a central processing unit (hereinafter, "CPU") 10, a random access memory (hereinafter "RAM") 20, a read only memory (hereinafter, "ROM") 30, and an electrically erasable programmable ROM (hereinafter, "E$^2$PROM") 40, as well as a timing control circuit 60. First scrambler circuits 11, 21, 31, and 41 are provided in data input and output (hereinafter, "I/O") sections or address I/O sections of the blocks 10, 20, 30, and 40 to be adjacent to the respective I/O sections. A bus line 50 connecting the first scrambler circuits 11, 21, 31, and 41 to one another is arranged. The timing control circuit 60 outputs a timing control signal at a predetermined timing. Each of the first scrambler circuits 11, 21, 31, and 41 changes connection of signals on the bus line 50 and scrambles the signals in response to this timing control signal. Namely, by performing scrambling in a time series manner, the analysis of information transmitted on the bus line 50 is made more difficult. On the outside of each of the first scrambler circuits 11, 21, 31, and 41 (bus line 50-side), the signals are scrambled so as to be replaced. On the inside thereof (each of memory RAM 20, ROM 30, and E$^2$PROM 40-sides), the signals are scrambled so as to be restored to original data.

As can be seen, according to the technique shown in Fig. 15, while the data is scrambled between the CPU 10 and each of the memories (RAM 20, ROM 30, and E'PROM 40), data on the memory is not scrambled. In other words, although the data on the bus line 50 can be protected, no measures are taken to protect the data on the memories or stored in the memories from being directly read and programmed.

Therefore, the conventional technique has disadvantages in that security measures are insufficiently taken to protect the intruder from probing the buses and the memories in the IC and reading or programming data, and from reading the data from each memory which is separated as a result of a separation analysis and decrypting original information.

SUMMARY OF THE INVENTION

The present invention has been achieved to solve the conventional disadvantages. It is an object of the present invention to provide a circuit capable of scrambling a signal transmitted on a bus, and capable of preventing any data on the bus and on a memory from being directly read and programmed and thereby preventing original information from being decrypted so as to store the scrambled data not only on the memory but also in the memory.

According to one aspect of the present invention, there is provided a scrambler circuit for converting to-be-processed data having four bits or more into processed data having as many bits as the to-be-processed data by predetermined scrambling, characterized in that the to-be-processed data is divided into a first data block having two bits or more and a second data block having as many bits as the first data block, and the processed data is divided into a third data block and a fourth data block each having as many bits as the first data block, and characterized in that the scrambler circuit comprises: a first scrambler unit that conducts predetermined first scrambling to the first data block, and that outputs first intermediate data having as many bits as the first data block; a first arithmetic unit that performs an exclusive OR operation between the second data block and the first intermediate data for each bit, and that outputs the third data block; a second scrambler unit that conducts one of the first scrambling and second scrambling different from the first scrambling to the third data block, and that outputs second intermediate data having as many bits as the third data block; and a second arithmetic unit that performs an exclusive OR operation between the second intermediate data and the first data block for each bit, and that outputs the fourth data block.

Further, the scrambler circuit according to the present invention is characterized in that each of the scrambler units converts input data into output data determined solely based on a conversion rule fixed to the each scrambler unit.

The scrambler circuit according to the present invention characterized as stated above can obtain processed data by scrambling to-be-processed data, and can prevent original information from being estimated. In addition, by appropriately setting the conversion rule for the scrambling performed by each of the first and second scrambler unit, a scrambling algorithm can be changed in a diversified manner, and security can be enhanced.

According to another aspect of the present invention, there is provided a descrambler circuit for inversely converting scrambled data having four or more bits into unprocessed data having as many bits as the scrambled data by predetermined descrambling, characterized in that the scrambled data is divided into a fifth data block having two bits or more and a sixth data block having as many bits as the fifth data block, and the ' unprocessed data is divided into a seventh data block and an eighth data block each having as many bits as the fifth data block, and characterized in that the descrambler circuit comprises: a third scrambler unit that conducts predetermined third scrambling to the fifth data block, and that outputs third intermediate data having as many bits as the fifth data block; a third arithmetic unit that performs an exclusive OR operation between the sixth data block and the third intermediate data for each bit, and that outputs the seventh data block; a fourth scrambler unit that conducts one of the third scrambling and fourth scrambling different from the third scrambling to the seventh data block, and that outputs fourth intermediate data having as many bits as the seventh data block; and a fourth arithmetic unit that performs an exclusive OR operation between the fourth intermediate data and the fifth data block for each bit, and that outputs the eighth data block.

Further, the descrambler circuit according to the present invention is characterized in that each of the scrambler units converts input data into output data determined solely based on a conversion rule fixed to the each scrambler unit.

The descrambler circuit according to the present invention characterized as stated above can inversely converts the scrambled data that is scrambled by the scrambler circuit according to the present invention into original, unprocessed data. The descrambler circuit according to the present invention, which is equal in circuit configuration to the scrambler circuit, uses the first scrambler unit in the scrambler circuit as the fourth scrambler unit, and the second scrambler unit in the scrambler unit as the third scrambler unit. It is thereby possible to simplify the configuration of the descrambler circuit.

It is preferable to constitute the scrambler circuit or the descrambler circuit according to the present invention such that one of the first and second scrambler units is constituted so that connection of part of or all of wirings between a plurality of input terminals corresponding to respective bits of the input data and a plurality of output terminals corresponding to respective bits of the output data is changed, and so that the conversion rule is fixed by change of the connection of the wirings. In this case, one of the first and second scrambler units conducts a cyclic shift operation to the input data by one bit or two or more bits, by the change of the connection of the wirings. Alternatively, one of the first and second scrambler units conducts a replacement operation to predetermined two bits of the input data by the change of the connection of the wirings. Alternatively, one of the first and second scrambler units conducts a combination of a cyclic shift operation to the input data by one bit or two or more bits and a replacement operation to predetermined two bits of the input data, by the change of the connection of the wirings. If the scrambler circuit or the descrambler circuit is constituted as stated finally, in particular, all combinations can be covered for the change of the connection of the wirings.

In the scrambler circuit or the descramble circuit according to the present invention, it is preferable that one of the first and second scrambler units includes a logic arithmetic circuit that conducts a predetermined logic operation to part of or all of the bits of the input data, and the conversion rule is fixed by the logic arithmetic circuit. In this case, the scrambler circuit or the descrambler circuit is constituted such that the logic arithmetic circuit conducts the logic operation to two bits or more of part of or all of the bits of the input data.

It is further preferable that the scrambler circuit or the descrambler circuit according to the present invention is constituted such that one of the first and second scrambler units includes a logic arithmetic circuit that conducts a predetermined logic operation to part of or all of the bits of the input data and to part of or all of bits of address data obtained when the input data is input, and the conversion rule is fixed by the logic arithmetic circuit so as to be determined solely based on an address value of the address data.

It is also preferable that the scrambler circuit or the descrambler circuit according to the present invention is constituted such that one of the first and second scrambler units includes a logic arithmetic circuit that conducts a predetermined logic operation to part of or all of the bits of the input data and to conversion rule fixing data stored in a predetermined nonvolatile memory, the conversion rule is fixed by the logic arithmetic circuit so as to be determined solely based on a data value of the conversion rule fixing data.

It is further preferable that the scrambler circuit or the descrambler circuit according to the present invention is constituted such that one of the first and second scrambler units comprises: a plurality of scrambler sub-units each of which converts the input data into the output data determined solely based on a conversion rule fixed in advance, the scrambler sub-units differing in the conversion rule; and a selection circuit that selects one of the output data of the plurality of scrambler sub-units, to which the same input data is input, and that outputs the selected output data based on a selection rule that changes according to predetermined information obtained when the input data is input, and such that the conversion rule fixed to each of the scrambler sub-units is fixed so as to be determined solely based on the scrambler sub-unit the output data of which is selected based on the selection rule. In this case, the scrambler circuit is preferably constituted such that one of the first and second scrambler units including the plurality of scrambler sub-units comprises: a code generation circuit that generates a selection code according to the predetermined information obtained when the input data is input, and that stores the selection code in a predetermined nonvolatile memory while making the selection code correspond to address data obtained when the input data is input; and a lookup table that makes the selection code correspond to each of the plurality of scrambler sub-units, and such that the selection circuit selects the output data from one of the output data of the plurality of scrambler sub-units, the selected output data being determined based on the selection code generated by the code generation circuit and the lookup table. In addition, the descrambler circuit is preferably constituted such that one of the third and fourth scrambler units comprising the plurality of scrambler sub-units comprises: a code read circuit that reads the selection code stored in a predetermined nonvolatile memory based on address data obtained when the input data is input to the plurality of scrambler sub-units; and a lookup table that makes the selection code correspond to each of the plurality of scrambler sub-units, and such that the selection circuit selects the output data from one of the output data of the plurality of scrambler sub-units, the selected output data being determined based on the selection code read by the code read circuit and the lookup table.

By adopting each of the constitution methods for the scrambler unit, it is possible to specify, in the form of hardware, a diversified conversion rule as a standard of the scrambling in the scrambler unit. This makes it either impossible or extremely difficult to decrypt data correlation the before and after the scrambling.

According to yet another aspect of the present invention, there is provided a data processing device characterized in that a plurality of functional blocks are connected to one another by an internal bus, and characterized by comprising the scrambler circuit according to the present invention provided in a first bus interface section between the internal bus and an external bus, the scrambler circuit inputting part of or all of data on the internal bus as the to-be-processed data, and outputting part of or all of data on the external bus as the processed data.

The data processing device according to the present invention characterized as stated above can transmit the data on the internal bus to the external bus after the data is scrambled, and store the data in, for example, an external storage device, thereby considerably enhancing data security.

According to still another aspect of the present invention, there is provided a data processing device, characterized in that a plurality of functional blocks are connected to one another by an internal bus, and characterized by comprising the descrambler circuit according to the present invention in a second bus interface section between the internal bus and the external bus, the descrambler circuit uses part of or all of data on the internal bus as the unprocessed data, and part of or all of data on the external bus as the scrambled.

The data processing device according to the present invention characterized as stated above receives the scrambled data that is scrambled by the scrambler circuit according to the present invention from the outside, and descrambles the data by the descrambler circuit according to the present invention, thereby making it possible to inversely convert the data into original, unprocessed data. It is, therefore, possible to make use of the original, unprocessed data on the internal bus while ensuring data security.

Further, the data processing device according to the present invention is characterized in that a plurality of functional blocks are connected to one another by an internal bus, and characterized by comprising: the scrambler circuit according to the present invention provided in a first bus interface section between the internal bus and an external bus, the scrambler circuit inputting part of or all of data on the internal bus as the to-be-processed data, and outputting part of or all of data on the external bus as the processed data; and a descramble circuit according to the present invention provided in a second bus interface section between the internal bus and the external bus, the descrambler circuit inputting part of or all of data on the internal bus as the to-be-processed data, and outputting part of or all of data on the external bus as the scrambled data.

The data processing device according to the present invention characterized as stated above can scramble the data on the internal bus, transmit the scrambled data to the external bus, and store the data in, for example, an external storage device, thereby considerably enhancing data security. In addition, the data processing device receives the scrambled data that is scrambled by the scrambler circuit according to the present invention from the outside and descrambles the data by the descrambler circuit, thereby inversely converting the data into original, unprocessed data. It is, therefore, possible to make use of the original, unprocessed data on the internal bus while ensuring data security

The scrambler circuit is not always equal to the scrambler circuit that scrambles the scrambled data to be descrambled by the descrambler circuit in the same data processing device. However, if they are equal, both the scrambling and the descrambling can be performed in the same data processing device. Therefore, operations for storing the scrambled data in the external storage device, reading the stored data, and reusing the data, and the like can be carried out.

The latter case, that is, case in which the descrambler circuit descrambles the scrambled data that is scrambled by the scrambler circuit in the same data processing device as that includes the descrambler circuit can be easily realized by using the first scrambler unit in the scrambler circuit as the fourth scrambler unit in the descrambler circuit and the second scrambler unit in the scrambler circuit as the third scrambler unit in the descrambler circuit.

Further, the data processing device according to the present invention is characterized in that the plurality of functional blocks are connected to one another by a second internal bus, and characterized by comprising the scrambler circuit according to present invention included in a third bus interface section between the second internal bus and a second external bus, the scrambler circuit inputting part of or all of data on the second internal bus as the to-be-processed data, and outputting part of or all of data on the second external bus as the processed data. The data processing device according to the present invention characterized as stated above can further enhance data security and data processing security.

In the data processing device according to the present invention, the internal bus and the external bus or the second internal bus and the second external bus may be divided into a plurality of blocks, each of the plurality of blocks comprising the scrambler circuit or the descrambler circuit.

Moreover, the data processing device according to the present invention is characterized in that the internal bus and the external bus are data buses and in that the second internal bus and the second external bus are address buses. The data processing device according to the present invention characterized as stated above includes the scrambler circuit that scrambles the data buses and the descrambler circuit that descrambles the data buses in the single data processing device, so that the data on the data buses and on the memories can be scrambled. In addition, since the data processing device includes the scrambler circuit that scrambles the address buses, the data can be protected more safely by accessing the memory using the scrambled address.

The data processing device according to the present invention is further characterized by being constituted as a semiconductor integrated circuit having the plurality of functional blocks and the bus interface section formed on a single semiconductor substrate. In addition, the data processing device functions as a one-chip microcomputer comprising an arithmetic logic unit as one of the functional blocks, and controlling the internal bus and the external bus. These features make it either impossible or extremely difficult for an intruder to probe the internal bus of the IC and the memories and to read or program the data, or to read the data from the memory separated as a result of a separation analysis and to decrypt original information.

According to still another aspect of the present invention, there is provided an IC card according to the present invention characterized by using the data processing device according to the present invention as a one-chip microcomputer for system control. The IC card according to the present invention characterized as stated above can scramble the data buses and the data on the memories, and thereby realize an IC card that ensures high security.

BRIEF DESCRIPTION OF THE DRAWINGS

Fig. 1 is a block diagram which illustrates one example of the internal configuration of a data processing device that includes a scrambler circuit and a descrambler circuit according to the present invention, according to one embodiment of the present invention;

Fig. 2 is a block diagram which illustrates one example of the internal configuration of the data processing device that includes the scrambler circuit and the descrambler circuit, according to another embodiment of the present invention;

Figs. 3A and 3B are block diagrams which illustrate the circuit configuration of the scrambler circuit and that of the descrambler circuit, respectively, according to one embodiment of the present invention;

Fig. 4 is a circuit block diagram which illustrates a scrambler unit, used in each of the scrambler circuit and the descrambler circuit according to the present invention, according to the first embodiment of the present invention;

Fig. 5 is a circuit block diagram which illustrates the scrambler unit, used in each of the scrambler circuit and the descrambler circuit according to the present invention, according to the second embodiment of the present invention;

Fig. 6 is a circuit block diagram which illustrates the scrambler unit, used in each of the scrambler circuit and the descrambler circuit according to the present invention, according to the fourth embodiment of the present invention;

Fig. 7 is a circuit block diagram which illustrates the scrambler unit, used in each of the scrambler circuit and the descrambler circuit according to the present invention, according to the fifth embodiment of the present invention;

Fig. 8 is a circuit block diagram which illustrates the scrambler unit, used in each of the scrambler circuit and the descrambler circuit according to the present invention, according to the sixth embodiment of the present invention;

Fig. 9 is a circuit block diagram which illustrates the scrambler unit, used in the scrambler circuit according to the present invention, according to the seventh embodiment of the present invention;

Fig. 10 is a circuit block diagram which illustrates the scrambler unit, used in the descrambler circuit according to the present invention, according to the eighth embodiment of the present invention;

Figs. 11A and 11B are explanatory views for data processing flows of the scrambler circuit and the descrambler circuit according to the present invention, respectively;

Fig. 12 is a block diagram which illustrates the scrambler circuit according to yet another embodiment of the present invention;

Fig. 13 is a block diagram which illustrates the descrambler circuit according to yet another embodiment of the present invention;

Fig. 14 is a block diagram which illustrates one example of the internal configuration of an IC card according to one embodiment of the present invention; and

Fig. 15 is an explanatory view for a conventional scrambling technique disclosed by a prior art publication.

## DETAILED DESCRIPTION OF THE INVENTION

Embodiments of a scrambler circuit, a descrambler circuit, and a data processing device which includes the scrambler circuit and the descrambler circuit according to the present invention will be described hereinafter with reference to the drawings.

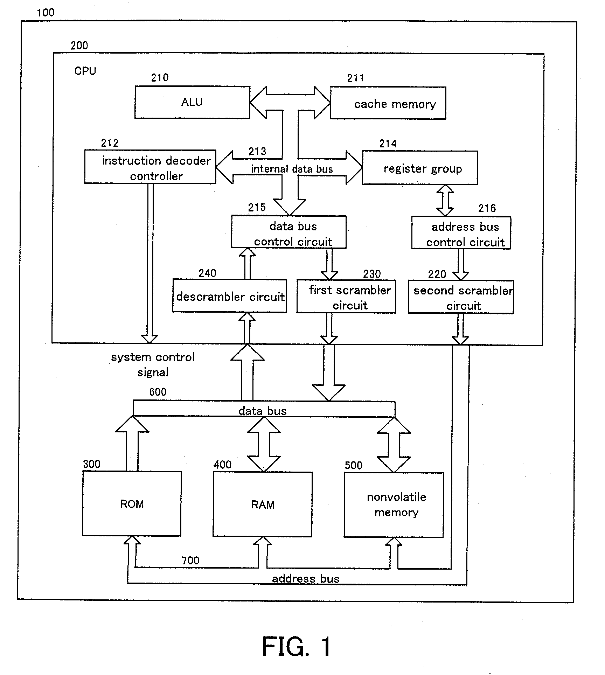

Fig. 1 illustrates one example of the internal configuration of a semiconductor integrated circuit 100 (hereinafter, "IC") that includes scrambler circuits 220 and 230 and a descrambler circuit 240 according to one embodiment of the present invention.

The IC 100 shown in Fig. 1 includes a CPU 200 that is one example of the data processing device according to the present invention, and includes, as external memories, a ROM 300 and a RAM 400 each connected to the CPU 200 by an external data bus 600 and an external address bus 700, and a nonvolatile memory 500 such as an $E^2PROM$. The IC 100 is constituted as a one-chip microcomputer.

In the CPU 200, an arithmetic logic unit (hereinafter, "ALU") 210, a cache memory 211, an instruction decoder controller 212, a register group 214, a data bus control circuit 215, and the like are connected to one another through an internal data bus 213. The register group 214 is connected to an address bus control circuit 216.

In the CPU 200, the first scrambler circuit 230 scrambles data on the internal data bus 213, and outputs the scrambled data to the external data bus 600. When data is input from the external data bus 600, the descrambler circuit 240 descrambles the input data, and transfers the descrambled data to the internal data bus 213.

When the CPU 200 accesses the external memory group, an address scrambled by the second scrambler circuit 220 is used. It is noted that the first scrambler circuit 230 and the second scrambler circuit 220 may be either equal or different in scrambling algorithm. In other words, the circuit configuration of a scrambler unit that fixes a conversion rule for each scrambler circuit to be described later may be either equal or different between the first and second scrambler circuits 220 and 230.

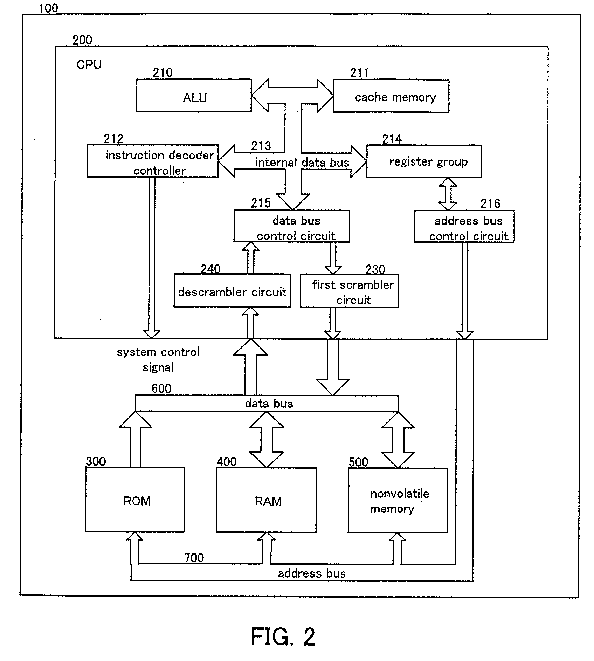

In the embodiment shown in Fig. 1, the address bus is scrambled. However, the scrambling of the address bus is not always essential. As shown in Fig. 2, therefore, the IC 100 may be constituted such that only the data bus is scrambled and such that no second scrambler circuit 220 is provided. The configuration shown in Fig. 2 is equal to that of the embodiment shown in Fig. 1 except that the second scrambler circuit 220 is not provided.

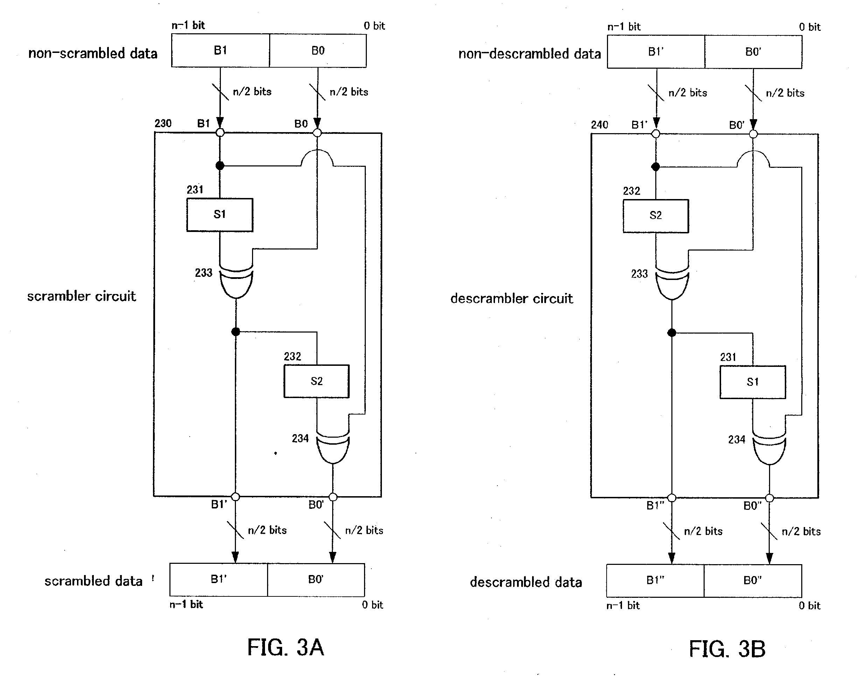

The circuit configurations of the scrambler circuits (first scrambler circuit 230 and second scrambler circuit 220) and the descrambler circuit 240 according to the present invention will next be described. Since the first scrambler circuit 230 and the second scrambler circuit 220 are equal in basic circuit configuration, one of the scrambler circuits will be described. Figs. 3A and 3B are circuit diagrams of the scrambler circuit 230 and the descrambler circuit 240, respectively.

As shown in Fig. 3A, non-scrambled data is divided into two data blocks of a first data block B1 (n/2 bits to (n-1) bits)) and a second data block B0 (0 bit to (n/2-1) bits), and the divided two data blocks B1 and B0 are input to the scrambler circuit 230. The scrambler circuit 230 outputs scrambled data having as many bits as the non-scrambled data and constructed by a third data block B1' (n/2 bits to (n-1) bits) and a fourth data block B0' (0 bit to (n/2-1) bits).

The scrambler circuit 230 includes a first scrambler unit 231 which subjects the input (first data block) B1 to first scrambling, a first arithmetic unit 233 which includes a plurality of exclusive OR circuits that perform an exclusive OR operation between an output (first intermediate data) of the first scrambler unit 231 and the input B0 for each bit, a second scrambler unit 232 which subjects third block data B1'that is an output of the first arithmetic unit 233 to second scrambling, and a second arithmetic unit 234 which includes a plurality of exclusive OR circuits that perform an exclusive OR operation between an output (second intermediate data) of the second scrambler unit 232 and the input B1 for each bit and that output the fourth data block B0'.

Likewise, as shown in Fig. 3B, non-descrambled scrambled data is divided into two data blocks of a fifth data block B1' (n/2 bits to (n-1) bits) and a sixth data block B0' (0 bit to (n/2-1) bits). The divided two data blocks B1' and B0' are input to the descrambler circuit 240. The descrambler circuit 240 outputs unprocessed data constructed by a seventh data block B1" (n/2 bits to (n-1) bits) and an eighth data block B0" (0 bit to (n/2-1) bits), having as many bits as the non-descrambled scrambled data, and inversely converted by descrambling before scrambling.

The descrambler circuit 240 includes a third scrambler unit 232 (equal to the second scrambler unit 232 in this embodiment) which subjects the input B1' to third scrambling (equal to the second scrambling in this embodiment), a third arithmetic unit 233 which includes a plurality of exclusive OR circuits which performs an exclusive OR operation between an output (third intermediate data) of the third scrambler unit 232 and the input B0' for each bit, a fourth scrambler unit 231 (equal to the first scrambler unit 231 in this embodiment) which subjects the seventh data block B1" that is an output of the third arithmetic unit 233 to fourth scrambling (equal to the first scrambling in this embodiment), and a fourth arithmetic unit 234 which includes a plurality of exclusive OR circuits that performs an exclusive OR operation between the an output (fourth intermediate data) of the fourth scrambler unit 231 and the input B1' for each bit, and that outputs the eighth data block B0".

The scrambling executed by the scrambler unit 231 and that executed by the scrambler unit 232 are constituted to convert the input data into output data determined solely by conversion rules fixed to the respective scrambler units.

It is noted, however, that the first scrambler unit 231 in the scrambler circuit 230 and the fourth scrambler unit 231 in the descrambler circuit 240 must be constituted to perform the same scrambling based on the same conversion rule. Likewise, the second scrambler unit 232 in the scrambler circuit 230 and the third scrambler unit 232 in the descrambler circuit 240 must be constituted to perform the same scrambling based on the same conversion rule. The first and fourth scrambler units 231 and the second and third scrambler units 232 may be either equal or different in configuration. However, if the units 231 and the units 232 are different in circuit configuration, it is possible to ensure more enhanced security. In addition, the first to fourth arithmetic units 233 and 234 are equal in circuit configuration.

While expressing an operation of the first (fourth) scrambler unit 231 as an S1 function and that of the second (third) scrambler unit 232 as an S2 function, operations of the scrambler circuit 230 and the descrambler circuit 240 will next be described.

The operation (scrambling) of the scrambler circuit 230 is expressed by the following Equations 1 and 2.

$$B1'=B0 \text{ xor } S1(B1) \qquad (1)$$

$$B0'=B1 \text{ xor } S2(B1') \qquad (2)$$

Next, the operation (descrambling) of the descrambler circuit 240 is expressed by the following Equations 3 and 4.

$$B1''=B0' \text{ xor } S2(B1') \qquad (3)$$

$$B0''=B1' \text{ xor } S1(B1'') \qquad (4)$$

Using Equations 1 to 4, the data scrambled by the scrambler circuit 230 is inversely converted by descrambling performed by the descrambler circuit 240, and returned to original data. Namely, if B0' in Equation 2 is assigned to B0' in Equation 3 to thereby delete B0', the following Equation 5 is obtained. Since an exclusive OR operation between multiple variables produces the same arithmetic result irrespective of their arithmetic orders, and an exclusive OR operation between the same values is zero, the following Equation 6 is obtained.

$$B1''=B1 \text{ xor } S2(B1') \text{ xor } S2(B1') \qquad (5)$$

$$B1''=B1 \text{ xor } 0=B1 \qquad (6)$$

Next, if B1' in Equation 1 is assigned to B1' in Equation 4 to delete B1', the following Equation 7 is obtained. Further, if B1" in Equation 6 is assigned to B1" in Equation 7 to delete B1", an exclusive OR operation between the multiple variables produces the same arithmetic result irrespective of their arithmetic orders and an exclusive OR operation between the same value is zero. Therefore, the following Equation 8 is obtained.

$$B0''=B0 \text{ xor } S1(B1) \text{ xor } S1(B1'') \qquad (7)$$

$$B0''=B0 \text{ xor } S1(B1) \text{ xor } S1(B1) =B0 \qquad (8)$$

It is thus demonstrated that the non-scrambled data B0 and B1 are equal to the descrambled data B0" and B1", respectively. In addition, the calculations can be made without depending on arithmetic contents of the functions S1 and S2. Therefore, as long as conditions that outputs of the functions S1 and S2 are determined solely relative to input arbitrary values are met, contents of the scrambling executed by the first and second scrambler units 231 and 232 can be arbitrarily selected. Accordingly, it suffices to select, as the functions S1 and S2, optimum processings in light of the trade-off between security enhancement and cost or feasibility such as circuit scale.

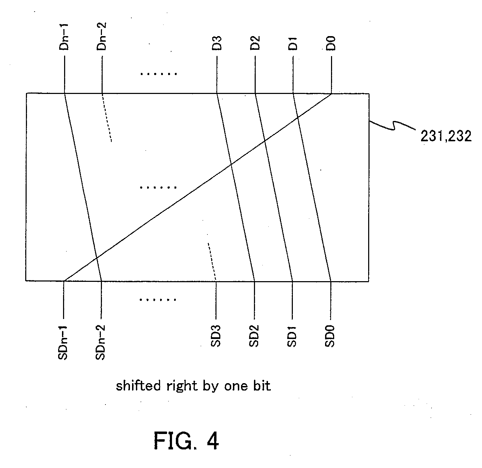

The circuit configuration of the first or second scrambler unit 231 or 232 for fixing the conversion rule that specifies the scrambling performed by the unit will next be described. Fig. 4 illustrates the circuit configuration of the first or second scrambler unit 231 or 232 according to the first embodiment.

As shown in Fig. 4, an output [SDn-1, SDn-2, ..., SD1, SD0] is shifted right by one bit relative to an input [Dn-1, Dn-2, ..., D1, D0]. As a result of this operation, the output [SDn-1, SDn-2, ..., SD1, SD0] is expressed as shown in the following Equation 9. It is assumed herein that D0 circulates and is shifted to a first bit on the left.

$$[SDn\text{-}1, SDn\text{-}2, ..., SD1, SD0]$$

$$=[D0, Dn\text{-}1,..., D2, D1] \qquad (9)$$

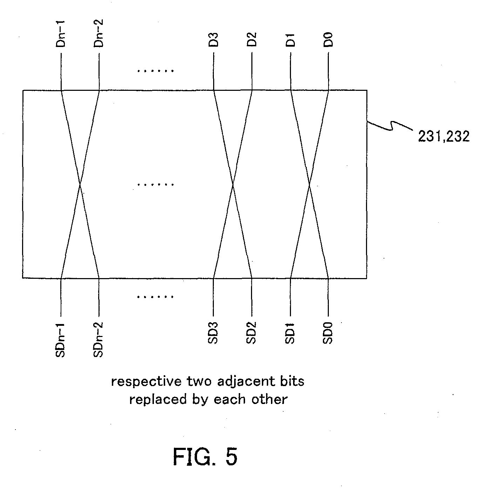

Fig. 5 illustrates the circuit configuration of the first or second scrambler unit 231 or 232 according to the second embodiment.

As shown in Fig. 5, respective two adjacent bits of the output [SDn-1, SDn-2, ..., SD1, SD0] are replaced by each other relative to the input [Dn-1, Dn-2, ..., D1, D0]. As a result of this operation, the output [SDn-1, SDn-2, ..., SD1, SD0] is expressed as shown in the following Equation 10.

$$[SDn\text{-}1, SDn\text{-}2, ..., SD1, SD0]$$

$$=[Dn\text{-}2, Dn\text{-}1,..., D0, D1] \qquad (10)$$

Although not shown in the drawing, as the circuit configuration of the first or second scrambler unit 231 or 232 according to the third embodiment, more diversified wiring replacement can be realized by an arbitrary combination of the scrambling in the first embodiment and that in the second embodiment.

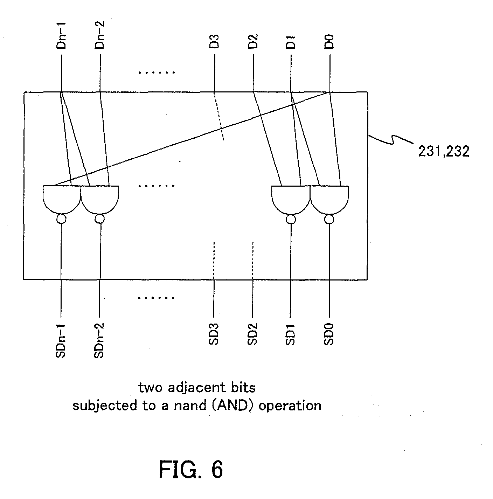

Fig. 6 illustrates the circuit configuration of the first or second scrambler unit 231 or 232 according to the fourth embodiment. In the first to third embodiments, the fixing of the conversion rule is realized by changing the wirings between a plurality of input terminals corresponding to respective bits of the input data and a plurality of output terminals corresponding to respective bits of the output data. In the fourth embodiment shown in Fig. 6, the fixing of the conversion rule is realized by subjecting each bit of the input data to a predetermined logic operation. Specifically, two adjacent bits of the output [SDn-1, SDn-2, ..., SD1, SD0] are subjected to a nand (AND) operation relative to the input [Dn-1, Dn-2, ..., D1, D0]. As a result of this operation, the output [SDn-1, SDn-2, ..., SD1, SD0] is expressed as shown in the following Equation 11.

$$[SDn\text{-}1, SDn\text{-}2, ..., SD1, SD0]$$

$$=[D0 \text{ nand } Dn\text{-}1,..., D1 \text{ nand } D0] \qquad (11)$$

The type of the AND operation is not limited to the nand operation, and the number of bits subjected to the operation may be arbitrarily changed.

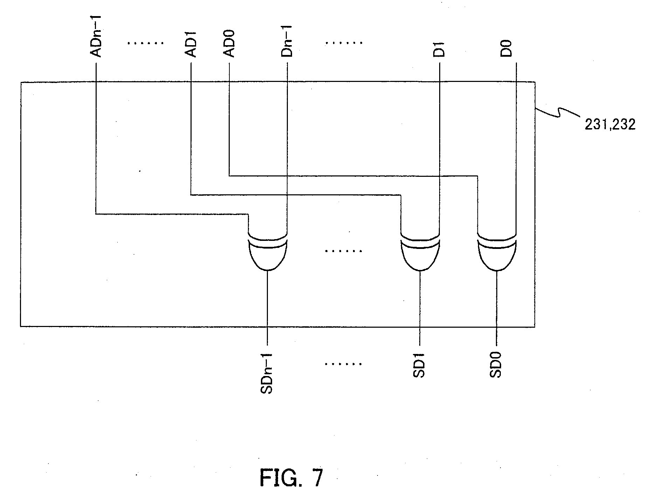

Fig. 7 illustrates the circuit configuration of the first or second scrambler unit 231 or 232 according to the fifth embodiment. In the first to fourth embodiment, the conversion rule for the conversion from the input data to the output data is always fixed irrespective of an address value of address data. In the fifth embodiment shown in Fig. 7, the input data is subjected to a logic operation using a memory address corresponding to the input data, whereby scrambling different among address values is realized.

Specifically, the output [SDn-1, SDn-2, ..., SD1, SD0] is obtained by performing an XOR (exclusive OR) operation between the input [Dn-1, Dn-2, ..., D1, D0] and the address [ADn-1, And-2, ..., AD1, AD0]. As a result of this operation, the output SD[n-1:0] is expressed as shown in the following Equation 12.

$$[SDn\text{-}1, SDn\text{-}2, ..., SD1, SD0]$$

$$=[Dn\text{-}1 \text{ xor } ADn\text{-}1, ..., D0 \text{ xor } AD0] \qquad (12)$$

As shown in Equation 12, the conversion rule that specifies the scrambling for an arbitrary address value is determined univocally. Therefore, even if the conversion rule is changed in the same scrambler units 231 and 232 according to the change of the address value, it is ensured that the data is inversely converted into non-scrambled data by using the same address value during inverse conversion. Namely, the address value functions as a key for determining the conversion rule that specifies the scrambling.

It is noted that the type of the logic operation is not limited to the exclusive OR operation, and that the number of bits of the key (address value in this embodiment) and the number of bits subjected to operation can be appropriately changed.

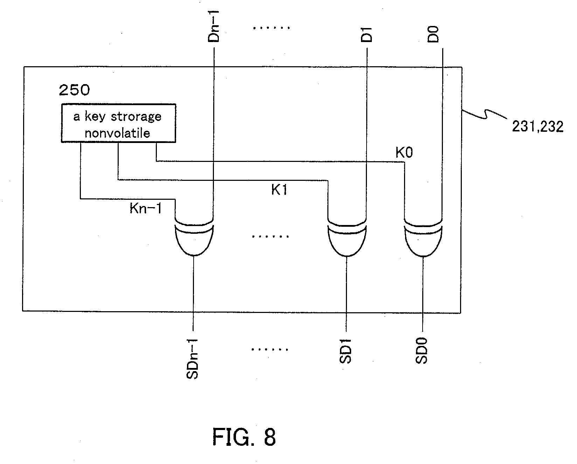

Fig. 8 illustrates the circuit configuration of the first or second scrambler unit 231 or 232 according to the sixth embodiment. In the first to fifth embodiments, as long as hardware configuration such as the replacement of wirings in the scrambler unit or the logic operation circuit, or the combination of the logic operation circuit and the address value is equal between the scrambler units, the conversion rule between the input data and the output data is constantly fixed. In the sixth embodiment shown in Fig. 8, the input data is subjected to a logic operation using key information (conversion rule fixing data) stored in a key storage nonvolatile memory 250. By doing so, even if the scrambler units are equal in hardware configuration or address value, different scrambling can be performed in the respective scrambler units.

Specifically, an xor (exclusive OR) operation is performed ,between the input [Dn-1, Dn-2, ..., D1, D0] and the key information [Kn-1, Kn-2, ..., Kn, K0] for each bit to obtain the output [SDn-1, SDn-2, ..., SD1, SD0]. As a result of this operation, the output SD[n-1:0] is expressed as shown in the following Equation 13.

$$[SDn\text{-}1, SDn\text{-}2, ..., SD1, SD0]$$

$$=[Dn\text{-}1 \text{ xor } Kn\text{-}1, ... , D0 \text{ xor } K0] \qquad (13)$$

The key information stored in the key storage nonvolatile memory 250 may be fixed while a device including the scrambler units is manufactured or may be set at an arbitrary value after manufacturing by writing means provided separately using a programmable nonvolatile memory.

The type of the logic operation is not limited to the exclusive OR operation, and the number of bits of the key information and the number of bits subjected to operation can be appropriately changed.

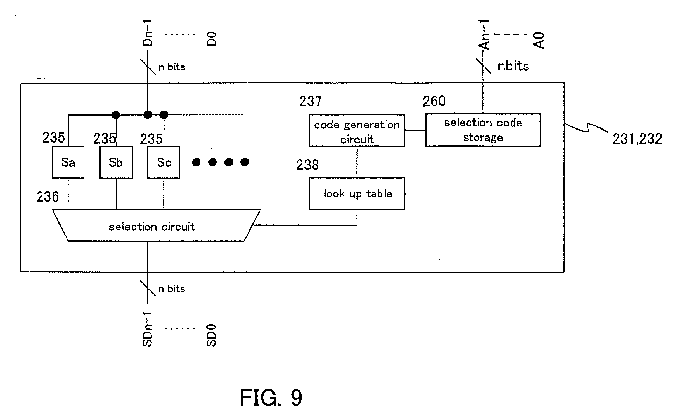

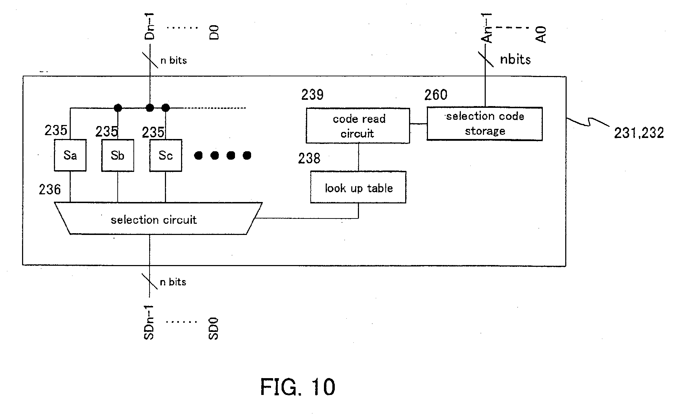

Figs. 9 and 10 illustrate the circuit configurations of the first (fourth) or second (third) scrambler unit 231 or 232 according to the seventh and eighth embodiments, respectively. In the first to sixth embodiments, the conversion rule for the conversion between the input data and the output data is always fixed in the same scrambler unit. In the seventh and eighth embodiments, a plurality of scrambler sub-units 235 having different conversion rules used for the input data are prepared. In addition, the scrambler unit 231 or 232 includes a selection circuit 236 which selects one of a plurality of pieces of output data (intermediate output data) that are as many as the scrambler sub-units 235, that are scrambled according to the conversion rules, and that are output from the scrambler sub-units 235, based on a selection rule that changes according to predetermined information obtained when the input data is input. With this constitution, the conversion rule fixed to the scrambler unit is sequentially changed according to the predetermined information obtained when the input data is input, whereby even the same scrambler unit realizes more complicated scrambling and descrambling. In other words, the conversion rule is not peculiar to the scrambler unit but is fixed solely according to the predetermined information obtained when the input data is input.

Each scrambler sub-unit 235 can be constituted by one of the scrambler units in the first to sixth embodiments having the circuit configuration of the first or second scrambler unit 231 or 232, or a new scrambler unit having a conversion rule obtained by combining two or more conversion rules of the scrambler units in the first to sixth embodiments.

The seventh embodiment is the embodiment in which the scrambler unit is limited to the first or second scrambler unit 231 or 232 employed in the scrambler circuit 230. The eighth embodiment is the embodiment in which the scrambler unit is limited to the third or fourth scrambler unit 232 or 231 employed in the descrambler circuit 240. The scrambler units in the first to sixth embodiments are the first and second scrambler units 231 and 232 in the scrambler circuit 230 and the fourth and third scramble units 231 and 232 in the descrambler circuit 240. Namely, the corresponding units are the same units. In the seventh and eighth embodiments, a location where each scrambler unit is used is fixed. However, the corresponding scrambler units in the seventh embodiment between the scrambler circuit 230 and the descrambler circuit 240 and those in the eighth embodiment are equal in fixed conversion rule. The respective circuits will be described in detail.

As shown in Fig. 9, the scrambler unit in the seventh embodiment includes the plural scrambler sub-unit 235, the selection circuit 236, and a code generation circuit 237 which generates a selection code according to the predetermined information obtained when the input data is input, and which stores the selection code and address data obtained when the input data is input in a selection code storage nonvolatile memory 260 while making them correspond to each other, and a lookup table 238 which makes each of the selection codes generated by the code generation circuit 237 to each of the scrambler sub-units 235. The nonvolatile memory 260 is shared between the scrambler unit in the seventh embodiment and the scrambler unit in the eighth embodiment to be described later. The nonvolatile memory 260 may be provided outside of the scrambler unit.

The code generation circuit 237 generates different selection code using random numbers or the like based on information on passage of time since the input of the input data, i.e., the start of the circuit and the address value of the address data. It is preferable that the number of generated selection codes is limited to the number of the scrambler sub-units 235. Even if the number of selection codes is not equal to the number of scrambler sub-units 235, no problem occurs as long as they can be made to correspond to one another in the lookup table 238. The code generation circuit 237 stores each generated selection code and the address value of the address data obtained when the selection code is generated in the nonvolatile memory 260. Alternatively, the code generation circuit 237 may store the generated selection code in an address area of the nonvolatile memory 260 that area corresponds to the address value of the address data obtained when the selection code is generated, in a one-on-one correspondence.

The lookup table 238 generates a selection instruction signal for instructing the selection of one scrambler sub-unit 235 corresponding to the selection code generated by the code generation circuit 237. The selection circuit 236 selects the intermediate output data from the selected scrambler sub-unit 235 based on the selection instruction signal, and outputs the selected intermediate output data as output data of the scrambler unit.

As shown in Fig. 10, the scrambler unit in the eighth embodiment includes the plural scrambler sub-units 235, the selection circuit 236, a code read circuit 239 which reads one selection code from the selection code storage nonvolatile memory 260, and the lookup table 238 which makes the codes as many as those generated in the scrambler unit in the seventh embodiment correspond to the plural scrambler sub-units 235, respectively. The nonvolatile memory 260 is shared between the scrambler unit in the eighth embodiment and the scrambler unit in the seventh embodiment. Therefore, the nonvolatile memory 260 may be provided outside of the scrambler unit.

The code read circuit 239 reads the selection code stored together with the address value from the nonvolatile memory 260 based on the address value of the address data obtained when the input data is input. Alternatively, the code read circuit 239 may read the selection code stored in the address area of the nonvolatile memory 260 corresponding to the address value, in a one-on-one correspondence.

The lookup table 238 generates a selection instruction signal for selecting one scrambler sub-unit 235 corresponding to the selection code read by the code read circuit 239. The selection code 236 selects the intermediate output data from one scrambler sub unit 235 based on the selection instruction signal, and outputs the selected intermediate output data as output data of the scrambler unit.

As the ninth embodiment of the first or second scrambler unit 231 or 232, although not shown in the drawing, the scrambler unit is preferably constituted to connect therein the respective sub-scrambler units to one another so that a plurality of scrambler sub-units arbitrarily selected from those in the scrambler units in the first to eighth embodiments are provided in one scrambler unit 231 or 232, input data of the scrambler unit in the ninth embodiment is input to at least one scrambler unit, output data of the scrambler unit in the ninth embodiment is output from at leas one scrambler sub-unit, and all of or part of the output data of at least one different scrambler sub-unit is input to at least one scrambler sub-unit. According to the configuration stated above, more complex and various scrambling can be performed.

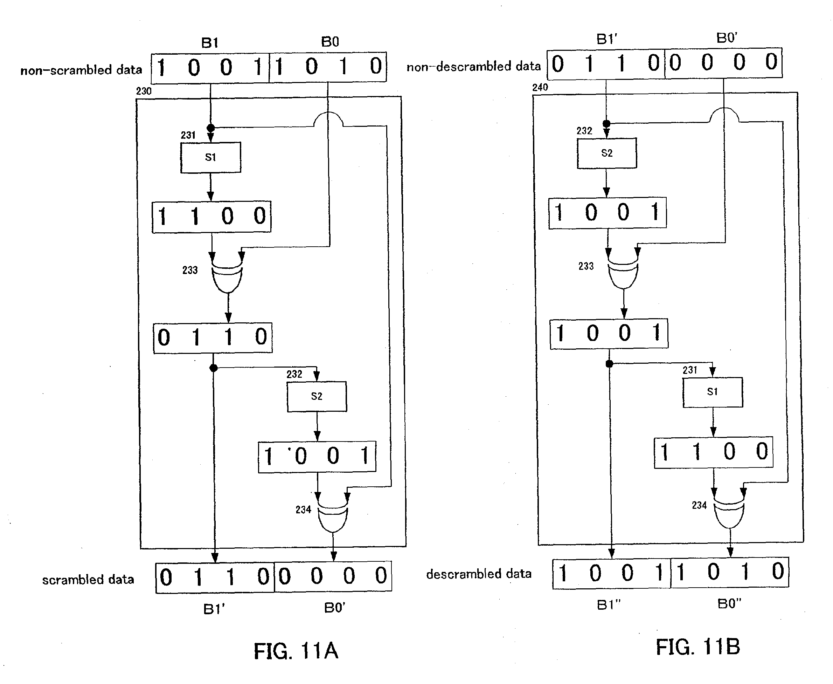

Figs. 11A and 11B illustrate processing flows of the scrambler circuit 230 and the descrambler circuit 240 using concrete numeric values, respectively. The first to fourth scrambler units 231 and 232 shown in Figs. 11A and 11B are such that the first and fourth scrambler units 231 have the circuit configuration in the first embodiment shown in Fig. 4 and that the second and third scrambler units 232 have the circuit configuration in the second embodiment shown in Fig. 5.

Fig. 11A illustrates the processing flow for the scrambling. As for original data "10011010", the first scrambler unit 231 shifts higher four bits "1001" right on a one-bit-by-one-bit basis to "1100". The first arithmetic unit 233 performs an exclusive OR operation between "1100" and lower four bits "1010" of the original data, and obtains "0110". Next, the second scrambler unit 232 replaces respective two adjacent bits of "0110" by each other, to obtain "1001". Finally, the second arithmetic unit 234 performs an exclusive OR operation between "1001" and "1001", and obtains "0000". As a result, scrambled data is "01100000".

Fig. 11B illustrates the processing flow for the descrambling. As for the scrambled data "01100000", the third (second) scrambler unit 232 replaces respective two adjacent bits of higher four bits "0110" by each other to obtain "1001". The third arithmetic unit 233 performs an exclusive OR operation between "1001" and lower four bits "0000" of the scrambled data, and obtains "1001". The fourth (first) scrambler unit 231 shifts "1001" right on a one-bit-by-one-bit basis, to "1100". Finally, the fourth arithmetic unit 234 performs an exclusive OR operation between "1100" and upper four bits "0110" of the scrambled data, and obtains "1010". As a result, descrambled data is "10011010", which coincides with the unscrambled, original data.

Another embodiment of the data processing device according to the present invention will be described.

<1> In the embodiments stated above, the data processing device is constituted to include one scrambler circuit 220 or 230 for the bus having a width of n bits, and one descrambler circuit 240 for the bus having a width of n bits. Alternatively, the data processing device may include two or more scrambler circuits 220 and 230 for the bus having the width of n bits and two or more descrambler circuits 240 for the bus having the width of n bits.

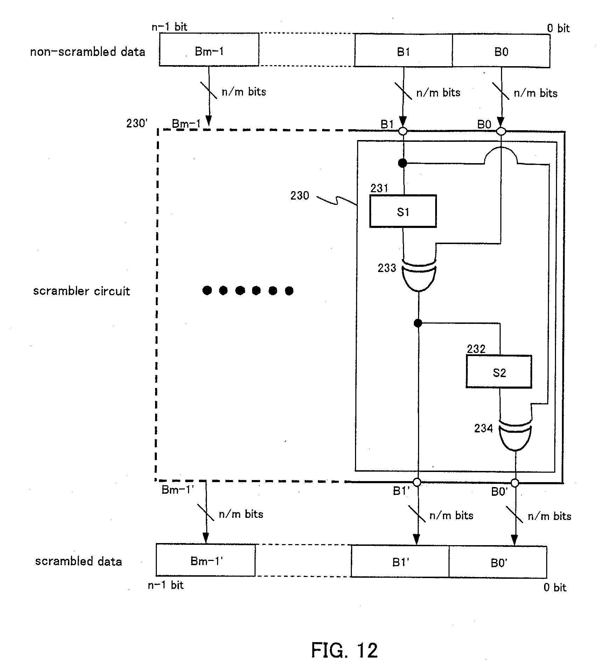

Fig. 12 illustrates one example of a scrambler circuit 230' when processing target data is divided into M data blocks. In this embodiment, M/2 scrambler circuits may be provided for each pair of two adjacent data blocks. In addition, by changing the first and second scrambler units 231 and 232 in each scrambler circuit 230 for each data block pair, it is possible to further enhance security.

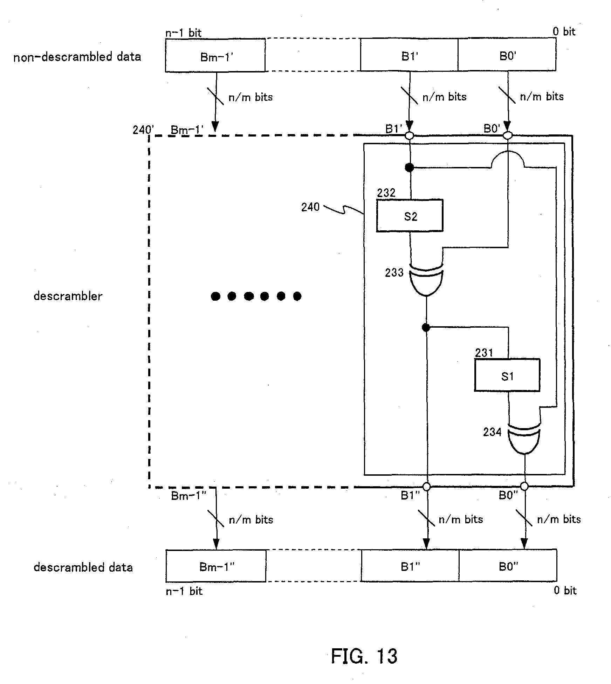

Likewise, Fig. 13 illustrates one example of a descrambler circuit 240' when the scrambled data is equally divided into M data blocks. In this embodiment, M/2 of descrambler circuits 240 may be provided for each pair of two adjacent data blocks.

<2> In the embodiments stated above, the CPU 200 includes the scrambler circuit 230 and the descrambler circuit 240 performing paired scrambling and descrambling. Alternatively, the CPU 200 may include only one of the scrambler circuit 230 and the descrambler circuit 240. Further, the descrambler circuit 240 may descramble the data scrambled by a scrambler circuit other than the scrambler circuit 230 included in the same CPU. In this case, the paired scrambling and descrambling are performed to be distributed in two or more data processing devices.

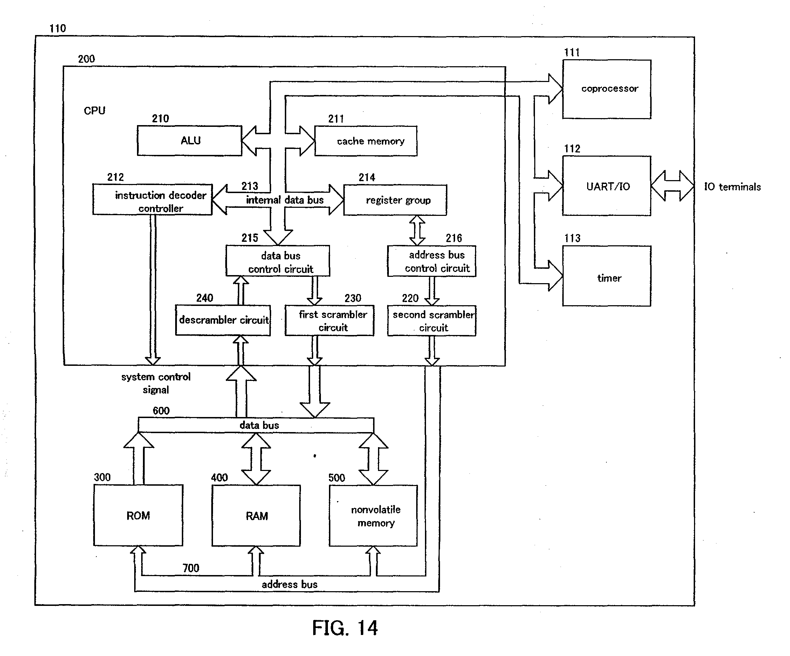

<3> Fig. 14 illustrates an example of the configuration when the data processing device according to the present invention is applied to an IC card.

The IC card 110 includes external memories such as the ROM 300, the RAM 400, and the nonvolatile memory 500 connected to the CPU 200 through the external data bus 600 and the external address bus 700, as well as a coprocessor 111, a UART/IO 112, and a timer 113. Normally, the IC card is required to ensure high security. A layout of the IC card is, therefore, elaborated. The constituent elements of the IC card 110 are laid out not as separate blocks but as one block on a semiconductor integrated circuit so as to prevent an intruder from specifying the locations of the CPU 200, the coprocessor 111, and an internal data bus 313 on the chip. Thanks to the microfabrication of a semiconductor manufacturing process, it is quite difficult to attack the internal data bus and the like provided as one block such as probing. An ordinary intruder, therefore, tries to probe a signal between the separate blocks such as the signal on the data bus between the CPU and the memories. However, by scrambling the data on the external data bus 600 between the separate blocks and the data in each memory, it is possible to provide the IC card having high security.

The IC card has been described as an applied embodiment of the present invention. The present invention can be applied versatilely to any device or system that processes secrete information such as individual information.

<4> In Figs. 1, 2, and 14, the examples in which the data processing device according to the present invention is a one-chip microcontroller as the semiconductor integrated circuit including the peripheral blocks such as the external memories are shown. However, as long as a plurality of functional blocks are connected to one another by the internal bus in the data processing device, and the scrambler circuit or the descrambler circuit according to the present invention is provided in the bus interface section between the internal bus and the external bus, it is not always necessary to form the data processing device and the peripheral blocks as the one-chip IC.

<5> In the embodiments stated above, it is assumed that the data processed by the scrambler circuit 230 and the descrambler circuit 240 has even bits and that the data bus widths of the internal data bus 213, the external data bus 600, and the like are even bits. Alternatively, if the bus width is odd bits, only one bit of the processing target bit may be excluded from the scrambling or descrambling target bits or a dummy one bit may be added to the processing target data to provide even bits.

<6> In the embodiments stated above, it is assumed that the data processed by the scrambler circuit 230 and the descrambler circuit 240 is parallel data. Alternatively, one of or all of the internal data bus 213, the external data bus 600, and the like may be serial buses. If serial data is processed, the data may be converted from the serial to parallel data, and input to the scrambler circuit 230 and the descrambler circuit 240 according to the present invention. The processing target data may be a combination of parallel data and serial data. If the internal bus has a width of eight bits and the external bus has a width of 16 bits, for example, then eight-bit data on the internal bus may be divided into two data blocks and the two divided data blocks may be read, the read data blocks may be scrambled by the scrambler circuit 230, and the scrambled 16-bit data may be transferred to the external bus.

As described so far in detail, the scrambler circuit, the descrambler circuit, and the data processing device according to the present invention scramble or descramble the data in the CPU. Namely, only the scrambled data is transmitted to the outside of the CPU through the data bus, and the external memories connected to this bus store the scrambled data. It is, therefore, possible to ensure quite high information secrecy against the probing of the signal on the external bus and the separation analysis conducted to memory components. Further, by scrambling even the address bus signal, it is possible to make it more difficult to analyze the signal. In the scrambler circuit or the descrambler circuit, a security processing (for making data secret) including not only the signal scrambling by the scrambler units but also the arithmetic processings of exclusive OR operations is performed. It is, therefore, possible to provide the data processing device capable of realizing high security enough to prevent the decryption of data, and capable of ensuring that original information can be logically restored.

Although the present invention has been described in terms of preferred embodiments, it will be appreciated that various modifications and alterations might be made by those skilled in the art without departing from the spirit and scope of the invention. The invention should, therefore, be measured in terms of the claims which follow.

## Claims

1. A scrambler circuit for converting to-be-processed data having four bits or more into processed data having as many bits as the to-be-processed data by predetermined scrambling, **characterized in that**

said to-be-processed data is divided into a first data block having two bits or more and a second data block having as many bits as said first data block, and said processed data is divided into a third data block and a fourth data block each having as many bits as said first data block, and

**characterized in that**

said scrambler circuit comprises:

a first scrambler unit that conducts predetermined first scrambling to said first data block, and that outputs first intermediate data having as many bits as said first data block;
a first arithmetic unit that performs an exclusive OR operation between said second data block and said first intermediate data for each bit, and that outputs said third data block;
a second scrambler unit that conducts one of said first scrambling and second scrambling different from the first scrambling to said third data block, and that outputs second intermediate data having as many bits as said third data block; and
a second arithmetic unit that performs an exclusive OR operation between said second intermediate data and said first data block for each bit, and that outputs said fourth data block.

2. The scrambler circuit according to claim 1, **characterized in that**

each of said scrambler units converts input data into output data determined solely based on a conversion rule fixed to the each scrambler unit.

3. The scrambler circuit according to claim 2, **characterized in that**

one of said first and second scrambler units is constituted so that connection of part of or all of wirings between a plurality of input terminals corresponding to respective bits of the input data and a plurality of output terminals corresponding to respective bits of the output data is changed, and so that said conversion rule is fixed by change of the connection of the wirings.

4. The scrambler circuit according to claim 3, **characterized in that**

one of said first and second scrambler units conducts a cyclic shift operation to said input data by one bit or two or more bits, by said change of the connection of the wirings.

5. The scrambler circuit according to claim 3, **characterized in that**

one of said first and second scrambler units conducts a replacement operation to predetermined two bits of said input data by said change of the connection of the wirings.

6. The scrambler circuit according to claim 3, **characterized in that**

one of said first and second scrambler units conducts a combination of a cyclic shift operation to said input data by one bit or two or more bits and a replacement operation to predetermined two bits of said input data, by said change of the connection of the wirings.

7. The scrambler circuit according to any one of claims 2 to 6, **characterized in that**

one of said first and second scrambler units includes a logic arithmetic circuit that conducts a predetermined logic operation to part of or all of the bits of the input data, and said conversion rule is fixed by said logic arithmetic circuit.

8. The scrambler circuit according to claim 7, **characterized in that**

said logic arithmetic circuit conducts the logic operation to two bits or more of part of or all of the bits of said input data.

9. The scrambler circuit according to claim 2, **characterized in that**

one of said first and second scrambler units includes a logic arithmetic circuit that conducts a predetermined logic operation to part of or all of the bits of the input data and to part of or all of bits of address data obtained when said input data is input, and said conversion rule is fixed by said logic arithmetic circuit so as to be determined solely based on an address value of said address data.

10. The scrambler circuit according to claim 2, **characterized in that**

one of said first and second scrambler units includes a logic arithmetic circuit that conducts a predetermined logic operation to part of or all of the bits of the input data and to conversion rule fixing data stored in a predetermined nonvolatile memory, said conversion rule is fixed by said logic arithmetic circuit so as to be determined solely based on a data value of said conversion rule fixing data.

11. The scrambler circuit according to claim 2, **characterized in that**

one of said first and second scrambler units comprises:

a plurality of scrambler sub-units each of which converts the input data into the output data determined solely based on a conversion rule fixed in advance, the scrambler sub-units differing in said conversion rule; and

a selection circuit that selects one of the output data of said plurality of scrambler sub-units, to which the same input data is input, and that outputs the selected output data based on a selection rule that changes according to predetermined information obtained when said input data is input, and **characterized in that**

said conversion rule fixed to each of said scrambler sub-units is fixed so as to be determined solely based on said scrambler sub-unit the output data of which is selected based on said selection rule.

12. The scrambler circuit according to claim 11, **characterized in that**

one of said first and second scrambler units including said plurality of scrambler sub-units comprises:

a code generation circuit that generates a selection code according to the predetermined information obtained when said input data is input, and that stores the selection code in a predetermined nonvolatile memory while making the selection code correspond to address data obtained when the input data is input; and

a lookup table that makes said selection code corresponds to each of said plurality of scrambler sub-units, and **characterized in that**

said selection circuit selects said output data from one of the output data of said plurality of scrambler sub-units, the selected output data being determined based on said selection code generated by said code generation circuit and said lookup table.

13. A descrambler circuit for inversely converting scrambled data having four or more bits into unprocessed data having as many bits as the scrambled data by predetermined descrambling, **characterized in that**

said scrambled data is divided into a fifth data block having two bits or more and a sixth data block having as many bits as said fifth data block, and said unprocessed data is divided into a seventh data block and an eighth data block each having as many bits as said fifth data block, and **characterized in that**

said descrambler circuit comprises:

a third scrambler unit that conducts predetermined third scrambling to said fifth data block, and that outputs third intermediate data having as many bits as said fifth data block;

a third arithmetic unit that performs an exclusive OR operation between said sixth data block and said third intermediate data for each bit, and that outputs said seventh data block;

a fourth scrambler unit that conducts one of said third scrambling and fourth scrambling different from the third scrambling to said seventh data block, and that outputs fourth intermediate data having as many bits as said seventh data block; and

a fourth arithmetic unit that performs an exclusive OR operation between said fourth intermediate data and said fifth data block for each bit, and that outputs said eighth data block.

14. The descrambler circuit according to claim 13, **characterized in that**

each of said scrambler units converts input data into output data determined solely based on a conversion rule fixed to the each scrambler unit.

15. The descrambler circuit according to claim 13, **characterized in that**

one of said third and fourth scrambler units is constituted so that connection of part of or all of wirings between a plurality of input terminals corresponding to respective bits of the input data and a plurality of output terminals corresponding to respective bits of the output data is changed, and so that said conversion rule is fixed by change of the connection of the wirings.

16. The descrambler circuit according to claim 15, **characterized in that**

one of said third and fourth scrambler units conducts a cyclic shift operation to said input data by one bit or two or more bits, by said change of the connection of the wirings.

17. The descrambler circuit according to claim 15, **characterized in that**

one of said third and fourth scrambler units conducts a replacement operation to predetermined two bits of said input data by said change of the connection of the wirings.

18. The descrambler circuit according to claim 15, **characterized in that**

one of said third and fourth scrambler units conducts a combination of a cyclic shift operation to said input data by one bit or two or more bits and a replacement operation to predetermined two bits of said input data, by said change of the connection of the wirings.

19. The descrambler circuit according to claim 14, **characterized in that**

one of said third and fourth scrambler units includes a logic arithmetic circuit that conducts a predetermined logic operation to part of or all of the bits of the input data, and said conversion rule is fixed by said logic arithmetic circuit.

20. The descrambler circuit according to claim 19, **characterized in that**
said logic arithmetic circuit conducts the logic operation to two bits or more of part of or all of the bits of said input data.

21. The descrambler circuit according to claim 14, **characterized in that**
one of said third and fourth scrambler units comprises a logic arithmetic circuit that conducts a predetermined logic operation to part of or all of the bits of the input data and to part of or all of bits of address data obtained when said input data is input, and said conversion rule is fixed by said logic arithmetic circuit so as to be determined solely based on an address value of said address data.

22. The descrambler circuit according to claim 14, **characterized in that**
one of said third and fourth scrambler units includes a logic arithmetic circuit that conducts a predetermined logic operation to part of or all of the bits of the input data and to conversion rule fixing data stored in a predetermined nonvolatile memory, said conversion rule is fixed by said logic arithmetic circuit so as to be determined solely based on a data value of said conversion rule fixing data.

23. The descrambler circuit according to claim 14, **characterized in that**
one of said third and fourth scrambler units comprises:

a plurality of scrambler sub-units each of which converts the input data into the output data determined solely based on a conversion rule fixed in advance, the scrambler sub-units differing in said conversion rule; and
a selection circuit that selects one of the output data of said plurality of scrambler sub-units, to which the same input data is input, and that outputs the selected output data based on a selection rule that changes according to predetermined information obtained when said input data is input, and **characterized in that**
said conversion rule fixed to each of said scrambler sub-units is fixed so as to be determined solely based on said scrambler sub-unit the output data of which is selected based on said selection rule.

24. The descrambler circuit according to 23, **characterized in that**

one of said third and fourth scrambler units comprising said plurality of scrambler sub-units comprises:

a code read circuit that reads the selection code stored in a predetermined nonvolatile memory based on address data obtained when said input data is input to said plurality of scrambler sub-units; and
a lookup table that makes said selection code corresponds to each of said plurality of scrambler sub-units, and **characterized in that**
said selection circuit selects said output data from one of the output data of said plurality of scrambler sub-units, the selected output data being determined based on said selection code read by said code read circuit and said lookup table.

25. A data processing device **characterized in that**
a plurality of functional blocks are connected to one another by an internal bus,
the scrambler circuit according to claim 1 is included in a first bus interface section between said internal bus and an external bus, and
said scrambler circuit inputs part of or all of data on said internal bus as said to-be-processed data, and outputs part of or all of data on said external bus as said processed data.

26. The data processing device according to claim 25, **characterized in that**
said internal bus and said external bus are divided into a plurality of blocks, each of said plurality of blocks comprising said scrambler circuit.

27. A data processing device, **characterized in that**
a plurality of functional blocks are connected to one another by an internal bus,
the descrambler circuit according to claim 13 is included in a second bus interface section between said internal bus and said external bus, and
said descrambler circuit inputs part of or all of data on said external bus as said scrambled data, and outputs part of or all of data on said internal bus as said unprocessed data that has been inversely converted.

28. The data processing device according to claim 25, **characterized in that**
the descrambler circuit according to claim 13 is included in a second bus interface section between said internal bus and said external bus, and
said descrambler circuit inputs part of or all of data on said external bus as said scrambled data, and outputs part of or all of data on said internal bus as said unprocessed data that has been inversely converted.

**29.** The data processing device according to claim 28, **characterized in that**

said first scrambler unit in said scrambler circuit and said fourth scrambler unit in said descrambler circuit perform equal scrambling based on an equal conversion rule, and

said second scrambler unit in said scrambler circuit and said third scrambler unit in said descrambler circuit perform equal scrambling based on an equal conversion rule.

**30.** The data processing device according to claim 27,

said internal bus and said external bus are divided into a plurality of blocks, each of said plurality of blocks comprising said descrambler circuit.

**31.** The data processing device according to claim 25, **characterized in that**

said internal bus and said external bus are data buses.

**32.** The data processing device according to claim 25, **characterized in that**

said plurality of functional blocks are connected to one another by a second internal bus,

the scrambler circuit according to claim 1 is included in a third bus interface section between said second internal bus and a second external bus, and

said scrambler circuit inputs part of or all of data on said second internal bus as said to-be-processed data, and outputs part of or all of data on said second external bus as said processed data.

**33.** The data processing device according to claim 32, **characterized in that**

said second internal bus and said second external bus are divided into a plurality of blocks, each of said plurality of blocks comprising said scrambler circuit.

**34.** The data processing device according to claim 32, **characterized in that**

said second internal bus and said second external bus are address buses.

**35.** The data processing device according to any one of claims 25 to 34, **characterized in that**

the data processing device is constituted as a semiconductor integrated circuit having said plurality of functional blocks and said bus interface section formed on a single semiconductor substrate.

**36.** The data processing device according to claim 35, **characterized in that**

the data processing device functions as a one-chip microcomputer comprising an arithmetic logic unit as one of said functional blocks, and controlling said internal bus and said external bus.

**37.** An IC card **characterized by** using the data processing device according to claim 36 as a one-chip microcomputer for system control.

100

200

CPU

| 210 | | 211 |
| ALU | | cache memory |

212
instruction decoder
controller

213
internal data bus

214
register group

215
data bus
control circuit

216
address bus
control circuit

240
descrambler circuit

230
first scrambler
circuit

220
second scrambler
circuit

system control
signal

600
data bus

| 300 | 400 | 500 |
| ROM | RAM | nonvolatile memory |

700
address bus

# FIG. 1

FIG. 2

n−1 bit     0 bit

non-scrambled data   | B1 | B0 |

n/2 bits    n/2 bits

230   B1     B0

231

S1

233

232

S2

234

scrambler circuit

B1'     B0'

n/2 bits    n/2 bits

scrambled data   | B1' | B0' |

n−1 bit     0 bit

FIG. 3A

---

n−1 bit     0 bit

non-descrambled data   | B1' | B0' |

n/2 bits    n/2 bits

240   B1'     B0'

232

S2

233

231

S1

234

descrambler circuit

B1''     B0''

n/2 bits    n/2 bits

descrambled data   | B1'' | B0'' |

n−1 bit     0 bit

FIG. 3B

shifted right by one bit

FIG. 4

respective two adjacent bits
replaced by each other

FIG. 5

two adjacent bits
subjected to a nand (AND) operation

FIG. 6

FIG. 7

FIG. 8

FIG. 9

FIG. 10

FIG. 11B

FIG. 11A

non-scrambled data

n-1 bit ... 0 bit

| Bm-1 | ... | B1 | B0 |

n/m bits   n/m bits   n/m bits

230'   Bm-1   B1   B0

scrambler circuit

• • • • • •

230

231
S1

233

232
S2

234

Bm-1'   B1'   B0'

n/m bits   n/m bits   n/m bits

scrambled data

| Bm-1' | ... | B1' | B0' |

n-1 bit   0 bit

FIG. 12

FIG. 13

FIG. 14

FIG. 15  PRIOR ART